Europäisches Patentamt

European Patent Office

Office européen des brevets

(19)

(11) Veröffentlichungsnummer : **0 202 610**
**B1**

# (12) EUROPÄISCHE PATENTSCHRIFT

(45) Veröffentlichungstag der Patentschrift :
28.09.88

(21) Anmeldenummer : 86106633.0

(22) Anmeldetag : 15.05.86

(51) Int. Cl.⁴ : **C 07 C 67/08, C 07 C 69/54**

(54) **Verfahren zur Herstellung von Alkylestern der alpha-beta-monoolefinisch ungesättigten Monocarbonsäuren.**

(30) Priorität : 23.05.85 DE 3518482

(43) Veröffentlichungstag der Anmeldung :
26.11.86 Patentblatt 86/48

(45) Bekanntmachung des Hinweises auf die Patenterteilung : 28.09.88 Patentblatt 88/39

(84) Benannte Vertragsstaaten :
BE DE FR GB IT NL

(56) Entgegenhaltungen :
DE-A- 2 548 561
FR-A- 2 196 992
FR-A- 2 316 253
US-A- 2 917 538
US-A- 4 330 643

(73) Patentinhaber : BASF Aktiengesellschaft
Carl-Bosch-Strasse 38
D-6700 Ludwigshafen (DE)

(72) Erfinder : Dietrich, Gerhard, Dr.
Stadtgartenstrasse 46
D-6700 Ludwigshafen (DE)
Erfinder : Nestler, Gerhard, Dr.
Van-Leyden-Strasse 17
D-6700 Ludwigshafen (DE)
Erfinder : Ruckh, Peter, Dr.
Karl-Valentin-Strasse 7
D-6800 Mannheim 51 (DE)
Erfinder : Herzog, Reinhard, Dr.
An der Froschlache 23
D-6700 Ludwigshafen (DE)

EP 0 202 610 B1

**Beschreibung**

Die vorliegende Erfindung betrifft ein Verfahren zur Herstellung von Alkylestern der α,β-monoolefinisch ungesättigten Carbonsäuren, insbesondere der Acryl- und Methacrylsäure durch Veresterung der Säuren mit 6 bis 20 C-Atome enthaltenden Alkoholen in Gegenwart einer starken Säure.

Für die großtechnische Herstellung von Acryl- und Methacrylsäurealkylestern bedient man sich im wesentlichen zweier Methoden, nämlich

1. der durch alkalisch wirkende Stoffe katalysierten Umesterung von Estern aus niederen Alkoholen und Acryl- oder Methacrylsäure, z. B. Acrylsäuremethyl- oder -ethylester, mit einem höhermolekularen Alkohol, z. B. 2-Ethylhexanol, wie es z. B. aus der US-PS 2 822 348, der GP-PS 960 005 oder der DE-AS 10 67 805 bekannt ist, oder

2. der durch starke Säuren katalysierten Veresterung der Acryl- oder Methacrylsäure mit Alkoholen, wie es z. B. in der US-PS 2 917 538 oder der DE-PS 25 48 561 beschrieben ist.

Bei den Verfahren der Umesterung wird der niedere Carbonsäurealkylester, z. B. der Acrylsäure- bzw. Methacrylsäureester im Überschuß über den höhermolekularen Alkohol eingesetzt und zur Abtrennung des entstehenden niederen Alkohols vom Reaktionsgemisch abdestilliert und nach Abtrennung des niederen Alkohols wieder in dieses zurückgeführt. Bei diesem Verfahren ist jedoch häufig die Abtrennung des niederen Alkohols sowie des eingesetzten alkalisch-wirkenden Katalysators schwierig, und das Verfahren hat daher in der Praxis keine große Bedeutung erlangt.

Bei der Umsetzung von α,β-monoolefinisch ungesättigten Carbonsäuren, wie Acrylsäure- bzw. Methacrylsäure, mit Alkoholen z. B. unter Bildung der Acrylsäure- bzw. Methacrylsäurealkylester handelt es sich um eine Gleichgewichtsreaktion. Der Umsetzungsgrad ist somit durch die Gleichgewichtskonstante bestimmt. Um einen hohen Umsatz zu erzielen, ist daher ein großer Überschuß an Monocarbonsäure oder Alkohol erforderlich, oder muß das bei der Umsetzung entstehende Wasser (Reaktionswasser) aus dem Reaktionsgemisch unter Zusatz einer Hilfsflüssigkeit durch azeotrope Destillation abgetrennt oder an einem zugesetzten Trocknungsmittel gebunden werden. Der Zusatz weiterer Einsatzstoffe, z. B. eines Schleppmittels, stellt jedoch einen erheblichen Nachteil dar, da diese durch aufwendige Destillation gereinigt werden müssen, um sie in den Prozeß zurückführen zu können.

So wird beispielsweise bei dem Verfahren der US-A-4 330 643 bei der Veresterung eines Polyesters mit eines α,β-olefinisch ungesättigten Carbonsäure unter azeotropem Abdestillieren des Reaktionswassers in Toluol gearbeitet, nachdem der Polyester zuvor z. B. aus Dimethylterephthalat, Ethylenglykol und Neopentylglykol unter Durchleiten von Stickstoff hergestellt wurde.

Nach dem Verfahren der DE-PS 25 48 561 kann nun das Verfahren der direkten Veresterung auch ohne zusätzliche Schleppmittel durch Umsetzung der Acryl- oder Methacrylsäure mit einem Überschuß an 2-Ethylhexanol in Gegenwart von 0,2 bis 5 Gew.% Schwefelsäure sowie eines Polymerisationsinhibitors, gegebenenfals in Kombination mit Luft, bei 85 bis 140 °C unter vermindertem Druck (50 bis 200 Torr) durchgeführt werden, wobei das im Überschuß eingesetzte 2-Ethylhexanol als Schleppmittel für das azeotrope Abdestillieren des Wassers dient. Bei dieser Verfahrensvariante ist vor allem von Nachteil, daß unter vermindertem Druck gearbeitet werden muß, was einen beachtlichen technischen Aufwand bedingt, und daß ein Überschuß an 2-Ethylhexanol eingesetzt werden muß, durch den die Bildung von Nebenprodukten, deren Abtrennung aufwendig ist, vermehrt wird. Als Nebenprodukte entstehen dabei hauptsächlich Ether und Olefine aus dem eingesetzten 2-Ethylhexanol sowie Additionsverbindungen aus der Acryl- oder Methacrylsäure oder deren Ester mit dem 2-Ethylhexanol. Dabei ist zur Abtrennung der Ether und Olefine eine technisch aufwendige Destillation erforderlich, um durch die notwendige Rückführung des überschüssigen 2-Ethylhexanols eine Anreicherung dieser Nebenprodukte zu verhindern. Die als Destillationsrückstand anfallenden Additionsprodukte müssen zudem verbrannt oder deponiert werden (vgl. den Vergleichsversuch gemäß DE-PS 25 48 561).

Es wurde nun gefunden, daß man Alkylester der α,β-monoolefinisch ungesättigten Monocarbonsäuren durch Veresterung der Monocarbonsäure mit 6 bis 20 C-Atomen enthaltenden Alkoholen in Gegenwart von 0,1 bis 5 Gew.%, bezogen auf das Reaktionsgemisch, starken Säuren sowie von Polymerisationsinhibitoren und einem Sauerstoff enthaltenden Gas bei 80 bis 150 °C mit Vorteil herstellen kann, wenn das molare Verhältnis von α,β-monoolefinisch ungesättigter Monocarbonsäure zu Alkohol 1 : 0,8 bis 1,2 beträgt und das Reaktionswasser aus dem Reaktionsgemisch unter Durchleiten eines 1 bis 20 Vol.% Sauerstoff enthaltenden Inertgases abgetrennt wird. Bei dem neuen Verfahren wird im allgemeinen unter atmosphärischem Druck, d. h. ohne Erhöhung oder Verminderung des Drucks, gearbeitet. Im allgemeinen wird dabei das Reaktionswasser und eine geringe Menge des Alkohols über Kopf einer dem Reaktionsgefäß aufgesetzten Destillationskolonne abdestilliert, wobei der abgeschiedene Alkohol dem Reaktionsgefäß über die Kolonne wieder zugeführt wird. Das Verfahren wird vorzugsweise kontinuierlich durchgeführt, wobei man vorteilhaft eine Kaskade von 2 oder mehr Reaktionsgefäßen verwendet, wobei dem ersten Reaktionsgefäß die α,β-monoolefinisch ungesättigte Monocarbonsäure und der Alkohol in einem Molverhältnis von 1 : 0,8 bis 0,98, und der restliche Alkohol den weiteren Reaktionsgefäßen zugeführt wird. Die Reaktions- bzw. Verweilzeit beträgt im allgemeinen 1 bis 20 Stunden, und die Aufarbeitung des Reaktionsprodukts kann in an sich üblicher Weise, insbesondere destillativ erfolgen.

Für das neue Verfahren werden als α,β-monoolefinisch ungesättigte Monocarbonsäuren die Acryl- und die Methacrylsäure vorgezogen. In Frage kommen ferner die Crotonsäure und α-Methylcrotonsäure. Derartige α,β-monoolefinisch ungesättigte Monocarbonsäuren haben 3 bis 5 C-Atome.

Als Alkohole, die gegebenenfalls substituiert sein können, kommen für das neue Verfahren besonders Alkanole und Alkandiole wie n-Hexandiol, n-Octanol, iso-Octanol, 2-Ethylhexanol, n-Decanol, Dodecylalkohol, Hexadecanol, Octadecanol sowie ferner als substituierte Alkanole Benzylalkohol, Phenylethylalkohol, Diethylenglykolmonoethylether und Tripropylenglykol in Betracht. Als starke Säuren werden bei dem neuen Verfahren meist Schwefelsäure oder para-Toluolsulfonsäure eingesetzt, doch kommen auch Benzolsulfonsäure, Naphtholsulfonsäuren und Methansulfonsäure in Betracht.

Geeignete Polymerisationsinhibitoren, die meist in Mengen von 100 bis 1 000 ppm, bezogen auf die α,β-monoolefinisch ungesättigte Carbonsäure, eingesetzt werden, sind z. B. Phenothiazin, Methylenblau, para-Benzophenon, Hydrochinon und Hydrochinonmonomethylether, und als Sauerstoff enthaltendes Inertgas hat sich vor allem Luft bewährt, die auch mit Stickstoff bis zu einem Sauerstoffgehalt von 1 Vol.% verdünnt sein kann. Das Sauerstoff enthaltende Inertgas soll im allgemeinen während der ganzen Veresterungsreaktion durch das Reaktionsgemisch geleitet werden. Im allgemeinen reichen hierfür 100 bis 600 Vol.-Teile des Inertgases je Teil Reaktionsgemisch und Stunde aus.

Das neue Verfahren hat den Vorteil, daß bei atmosphärischem Druck ohne nennenswerten Überschuß an Alkohol und ohne Mitverwendung zusätzlicher Schleppmittel gearbeitet werden kann, und daß dabei die Bildung von Nebenprodukten weitgehend unterdrückt wird. Aufgrund der stabilisierenden Wirkung des Sauerstoffs kann zudem auf eine zusätzliche Stabilisierung in den Destillationskolonnen oft verzichtet werden. Das neue Verfahren ist daher besonders einfach und Energie-sparend.

Die in den folgenden Beispielen angegebenen Teile und Prozente beziehen sich auf das Gewicht. Die darin angegebenen Volumenteile verhalten sich zu den Gewichtsteilen wie das Liter zum Kilogramm.

Beispiel 1

Apparatur : 2 hintereinander geschaltete Umlaufreaktoren mit einem Füllvolumen von jeweils 1 000 Volumenteilen und aufgesetzten Destillationskolonnen mit Wasserabscheidern.

Im ersten Umlaufreaktor werden je Stunde 190 Teile Methacrylsäure, 5 Teile Schwefelsäure und 0,3 Teile Phenothiazin direkt und 280 Teile n-Octanol über dessen Destillationskolonne zugeführt. Das molare Verhältnis von Methacrylsäure zu Octanol beträgt 1 : 0,97.

Dem zweiten Reaktor werden zusätzlich zu dem Austrag des ersten Reaktors je Stunde 36 Teile n-Octanol über dessen Destillationskolonne zugeführt. Durch die Reaktionsgemische in beiden Reaktoren werden je Stunde jeweils 150 000 Vol.-Teile Luft geleitet. Die Reaktionstemperatur beträgt jeweils 120 °C. Über die beiden Wasserabscheider werden je Stunde zusammen 40 Teile Wasser abgetrennt. Der Reaktoraustrag beträgt 470 Teile je Stunde. Er enthält 90,5 % n-Octylmethacrylat, 0,7 % Methacrylsäure, 5,8 % Octanol, 0,5 % hochsiedende Nebenprodukte und Spuren an Dioctylether und Olefinen. Der Umsatz, bezogen auf Methacrylsäure beträgt 98,7 Mol.%, bezogen auf Octanol 91,4 Mol.% und die Selektivität, bezogen auf Methacrylsäure 98,2 Mol.%, bezogen auf Octanol 97,3 Mol.%.

Beispiel 2

In einer Apparatur, wie sie in Beispiel 1 angegeben ist, werden dem ersten Umlaufreaktor je Stunde 172 Teile Methacrylsäure, 5 Teile Schwefelsäure und 0,3 Teile Phenothiazin direkt und 250 Teile 2-Ethylhexanol über die Destillationskolonne zugeführt. Das Molverhältnis Methacrylsäure zu 2-Ethylhexanol beträgt 1 : 0,96. Dem zweiten Umlaufreaktor werden je Stunde 36 Teile 2-Ethylhexanol über dessen Destillationskolonne zugeführt. Durch die beiden Reaktionsgemische in den Umlaufreaktoren werden jeweils 150 000 Vol.-Teile Luft je Stunde geleitet. Die Reaktionstemperatur beträgt in beiden Reaktoren 120 °C. Über die beiden Wasserabscheider werden je Stunde zusammen 35 Teile Wasser abgetrennt. Der Reaktoraustrag von 427 Teilen je Stunde enthält 90,1 % 2-Ethylhexylmethacrylat, 0,7 % Methacrylsäure, 6,9 % 2-Ethylhexanol, 0,8 % hochsiedende Nebenprodukte und Spuren an Dioctylether und Olefinen. Der Umsatz an Methacrylsäure beträgt 98,2 Mol.%, an 2-Ethylhexanol 97,5 % und die Selektivität, bezogen auf Methacrylsäure 99,2 %, bezogen auf 2-Ethylhexanol 98,8 Mol.%.

Beispiel 3

Man arbeitet in einer Apparatur, wie sie in Beispiel 1 angegeben ist, und führt dem ersten Umlaufreaktor je Stunde 155 Teile Acrylsäure, 5 Teile Schwefelsäure (96 %ig) und 0,3 Teile Phenothiazin direkt zugeführt und über die Destillationskolonne zusätzlich 2 707 Teile 2-Ethylhexanol eingebracht. Im zweiten Reaktor führt man zusätzlich zu dem Austrag des ersten Umlaufreaktors über dessen Destillationskolonne je Stunde 40 Teile 2-Ethylhexanol zu. Das molare Verhältnis von Acrylsäure zu 2-Ethylhexanol beträgt demnach im ersten Umlaufreaktor 1 : 0,97. Durch beide Reaktionsgemische werden je Stunde jeweils 150 000 Vol.-Teile Luft geleitet. Beide Umlaufreaktoren werden auf 120 °C gehalten. Über die beiden Wasserabscheider werden je Stunde zusammen 38 Teile Wasser abgetrennt. Der Austrag des zweiten Umlaufreaktors (430 Teile je Stunde) enthält neben Stabilisator und Katalysator 87,4 % 2-Ethylhexylacrylat, 1,1 % Acrylsäure, 6,1 % 2-Ethylhexanol, 0,3 % Octene, 3,3 % hochsiedende Nebenprodukte und Spuren an Dioctylethern (weniger als 0,01 %). Der Umsatz an Acrylsäure beträgt 97 Mol.% an 2-Ethylhexanol 91,5 Mol.% und die Selektivität, bezogen auf die Acrylsäure, 97,7 %, bezogen auf 2-Ethylhexanol 93,5 Mol.%.

Beispiel 4

Man arbeitet in einer Apparatur, wie sie in Beispiel 1 angegeben ist und führt dem ersten Umlaufreaktor je Stunde 165 Teile Methacrylsäure, 5 Teile Schwefelsäure und 0,5 Teile Phenothiazin direkt und über dessen Destillationskolonne 350 Teile n-Dodecanol zu. Das molare Verhältnis von Methacrylsäure zu Dodecanol beträgt im Reaktor 1 : 0,98. Dem zweiten Umlaufreaktor werden zusätzlich zu dem Austrag des ersten Reaktors je Stunde über dessen Destillationskolonne 40 Teile Dodecanol zugeführt. Außerdem werden durch die Reaktionsgemische in beiden Reaktoren je Stunde jeweils 150 000 Vol.-Teile Luft geleitet. Die Reaktionstemperatur beträgt jeweils 120 °C. Über die beiden Wasserabscheider werden je Stunde zusammen 34 Teile Wasser abgetrennt. Der Reaktoraustrag (525 Teile je Stunde) enthält 89,7 % Dodecanol, 0,5 % Methacrylsäure, 7,7 % Dodecylalkohol, 0,9 % hochsiedende Nebenprodukte und Spuren an Ether und Olefinen. Der Umsatz, bezogen auf Methacrylsäure, beträgt 98,4 Mol.%, bezogen auf Dodecanol 89,5 Mol.% und die Selektivität, bezogen auf Methacrylsäure 98,5 %, bezogen auf Dodecanol 98,2 Mol.%.

Vergleichsversuch gemäß DE-PS 25 48 561

Einem Umlaufreaktor eines Füllvolumens von 1 000 Volumenteilen mit aufgesetzter Destillationskolonne und Wasserabscheider führt man je Stunde 60 Teile Acrylsäure, 2,2 Teile Schwefelsäure und 0,1 Teile eines Gemisches aus gleichen Teilen, Phenothiazin und Hydrochinon direkt und über dessen Destillationskolonne 154 Teile 2-Ethylhexanol zu. Das molare Verhältnis von Acrylsäure zu 2-Ethylhexanol beträgt 1 : 1,42. Die Veresterungsreaktion wird bei 119 °C unter vermindertem Druck (130 mbar) und einer Verweilzeit von 4 Stunden durchgeführt. Über den Wasserabscheider werden je Stunde 15 Teile Wasser abgetrennt. Der Reaktoraustrag von 201 Teilen je Stunde enthält neben den Stabilisatoren und Katalysator 70,8 % Ethylhexylacrylat, 20,6 2-Ethylhexanol, 0,6 % Acrylsäure, 0,1 % Dioctylether, 0,6 % Octene und 6,3 % hochsiedende Nebenprodukte. Der Umsatz, bezogen auf Acrylsäure, beträgt 98 Mol.%, bezogen auf 2-Ethylhexanol 71 Mol.% und die Selektivität, bezogen auf Acrylsäure 95 Mol.%, bezogen auf 2-Ethylhexanol 91,5 Mol.%.

Beispiel 5

In einer Apparatur, wie sie in Beispiel 1 angegeben ist, werden dem ersten Vorlaufreaktor je Stunde 158 Teile Acrylsäure, 5 Teile p-Toluolsulfonsäure und 0,5 Teile Hydrochinonmonomethylether direkt und 118 Teile 1,6-Hexandiol über die Destillationskolonne zugeführt. Das Äquivalentverhältnis Acrylsäure zu Hexandiol beträgt 1 : 0,91. Durch die Reaktionsgemische in den beiden Vorlaufreaktoren werden jeweils 150 000 Vol.-Teile Luft je Stunde geleitet. Die Temperatur beträgt in beiden Reaktoren 110 °C. Über die beiden Wasserabscheider werden je Stunde zusammen 35 Teile Wasser abgetrennt. Der Reaktionsaustrag von 246 Teilen je Stunde enthält 88,9 % 1,6-Hexandioldiacrylat. Der Umsatz an Acrylsäure beträgt 91,7 %, an Hexandiol 100 % und die Selektivität, bezogen auf Acrylsäure 95,6 %, bezogen auf Hexandiol 96,5 %.

**Patentanspruch**

Verfahren zur Herstellung von Alkylestern der α,β-monoolefinisch ungesättigten Monocarbonsäuren durch Veresterung der Carbonsäure mit 6 bis 20 C-Atome enthaltenden Alkoholen in Gegenwart von 0,1 bis 5 Gew.-%, bezogen auf das Reaktionsgemisch, starken Säuren sowie von Polymerisationsinhibitoren und einem Sauerstoff enthaltenden Gas bei 80 bis 150 °C, dadurch gekennzeichnet, daß das molare Verhältnis von α,β-olefinisch ungesättigter Monocarbonsäure zu Alkohol 1 : 0,8 bis 1,2 beträgt und das Reaktionswasser aus dem Reaktionsgemisch unter Durchleiten eines 1 bis 20 Vol.% Sauerstoff enthaltenden Inertgases abgetrennt wird.

**Claim**

A process for the preparation of an alkyl ester of α,β-monoolefinically unsaturated monocarboxylic acid by esterifying the carboxylic acid with an alcohol of 6 to 20 carbon atoms in the presence of from 0.1 to 5 % by weight, based on the reaction mixture, of a strong acid and in the presence of a polymerization inhibitor and an oxygen-containing gas, at from 80 to 150 °C, wherein the molar ratio of the α,β-olefinically unsaturated monocarboxylic acid to the alcohol is from 1 : 0.8 to 1 : 1.2, and the water of reaction is separated off from the reaction mixture by passing an inert gas containing from 1 to 20 % by volume of oxygen through the reaction mixture.

**Revendication**

Procédé de préparation d'esters alkyliques d'acides monocarboxyliques α,β-mono-oléfiniquement insaturés par estérification des acides carboxyliques avec des alcools contenant de 6 à 20 atomes de carbone, en présence de 0,1 à 5 % en poids, sur la base du mélange réactionnel, d'acides forts, ainsi que d'inhibiteurs de polymérisation et d'un gaz contenant de l'oxygène, à 80-150 °C caractérisé en ce que le rapport molaire des acides monocarboxyliques $\alpha,\beta$-oléfiniquement insaturés aux alcools s'élève à 1 : 0,8-1,2 et en ce qu'on sépare l'eau de réaction du mélange réactionnel en faisant passer un gaz inerte qui contient de 1 à 20 % en volume d'oxygène.